# EUROPEAN PATENT SPECIFICATION

(11) **EP 2 703 480 B1**
(45) Date of publication and mention of the grant of the patent: **29.08.2018**
(21) Application number: 12777460.2
(22) Date of filing: 19.04.2012
(51) Int. Cl.: C12N 5/074, C12N 5/0735, C12N 5/10, A61K 35/545

(54) **METHOD FOR PREPARING INDUCED PLURIPOTENT STEM CELLS USING MICROVESICLES DERIVED FROM EMBRYONIC STEM CELLS**
VERFAHREN ZUR HERSTELLUNG INDUZIERTER PLURIPOTENTER STAMMZELLEN MIT MIKROVESIKELN AUS EMBRYONALEN STAMMZELLEN
PROCÉDÉ DE PRÉPARATION DE CELLULES SOUCHES PLURIPOTENTES INDUITES À L'AIDE DE MICROVÉSICULES ISSUES DE CELLULES COUCHES EMBRYONNAIRES

(30) Priority: 28.04.2011 KR 20110040202
(43) Date of publication of application: 05.03.2014
(73) Proprietor: Postech Academy-Industry Foundation, Gyeongsangbuk-do 790-784 (KR)
(72) Inventor: PARK, Jae-Sung, Pohang-si Gyeongsangbuk-do 790-751 (KR); GHO, Yong Song, Pohang-si Gyeongsangbuk-do 790-751 (KR); KIM, Yoon Keun, Pohang-si Gyeongsangbuk-do 790-751 (KR); KIM, Jun Ho, Pohang-si Gyeongsangbuk-do 790-784 (KR); JANG, Su Chul, Yecheon-gun Gyeongsangbuk-do 757-910 (KR); YI, Namwoo, Seoul 136-772 (KR); JEONG, Dayeong, Ulsan 680-750 (KR); CHOI, Eun-Jeong, Busan 612-850 (KR)
(74) Representative: Dehns
(86) International application number: PCT/KR2012/003019
(87) International publication number: WO 2012/148130

(56) References cited:
- EP-A2- 2 450 032
- WO-A1-2013/158258
- KR-A- 20110 002 443
- KR-A- 20110 002 443
- RATAJCZAK J ET AL: "Embryonic stem cell-derived microvesicles reprogram hematopoietic progenitors: evidence for horizontal transfer of mRNA and protein delivery", LEUKEMIA, MACMILLAN PRESS LTD, US, vol. 20, no. 5, 1 May 2006 (2006-05-01), pages 847-856, XP002456317, ISSN: 0887-6924, DOI: 10.1038/SJ.LEU.2404132
- SAIYONG ZHU ET AL: "Reprogramming of Human Primary Somatic Cells by OCT4 and Chemical Compounds", CELL STEM CELL, vol. 7, no. 6, 1 December 2010 (2010-12-01), pages 651-655, XP055075780, ISSN: 1934-5909, DOI: 10.1016/j.stem.2010.11.015
- YUAN ALEX ET AL: "Transfer of MicroRNAs by Embryonic Stem Cell Microvesicles", PLOS ONE, PUBLIC LIBRARY OF SCIENCE, US, vol. 4, no. 3, 6 March 2009 (2009-03-06), pages e4722-1, XP002562228, ISSN: 1932-6203
- FEDERICA COLLINO ET AL: "Microvesicles Derived from Adult Human Bone Marrow and Tissue Specific Mesenchymal Stem Cells Shuttle Selected Pattern of miRNAs", PLOS ONE, vol. 5, no. 7, 27 July 2010 (2010-07-27), page e11803, XP055067748, DOI: 10.1371/journal.pone.0011803
- Diana Katsman: "Activation of the retina's regenerative potential by embryonic stem cell-derived microvesicles", , 1 January 2012 (2012-01-01), XP055168940, Retrieved from the Internet: URL:https://escholarship.org/uc/item/4p67r 7m7 [retrieved on 2015-02-11]
- DAYEONG JEONG ET AL: "Nanovesicles engineered from ES cells for enhanced cell proliferation", BIOMATERIALS, vol. 35, no. 34, 1 November 2014 (2014-11-01), pages 9302-9310, XP055168790, ISSN: 0142-9612, DOI: 10.1016/j.biomaterials.2014.07.047
- DIANA KATSMAN ET AL: "Embryonic Stem Cell-Derived Microvesicles Induce Gene Expression Changes in Müller Cells of the Retina", PLOS ONE, vol. 7, no. 11, 30 November 2012 (2012-11-30), page e50417, XP055168844, DOI: 10.1371/journal.pone.0050417
- RATAJCZAK ET AL.: 'Embryonic stem cell-derived microvesicles reprogram hematopoietic progenitors: evidence for horizontal transfer of mRNA and protein delivery' LEUKEMIA vol. 20, no. 5, 02 February 2006, pages 847 - 856, XP002456317
- YUAN ET AL.: 'Transfer of microRNAs by embryonic stem cell microvesicles' PLOS ONE vol. 4, no. 3, 06 March 2009, pages 1 - 8, XP002562228
- COLLINO ET AL.: 'Microvesicles derived from adult human bone marrow and tissue specific mesenchymal stem cells shuttle selected pattern of miRNAs' PLOS ONE vol. 5, no. 7, 27 July 2010, pages 1 - 15, XP055067748
- ZHU ET AL.: 'Reprogramming of human primary somatic cells by Oct4 and Chemical compounds' CELL STEM CELL vol. 7, no. 6, 03 December 2010, pages 651 - 655, XP055075780

## Description

The present invention relates to a method of preparing induced pluripotent stem cells by dedifferentiating somatic cells using embryonic stem cell-derived microvesicles.

Dedifferentiation is a process in which mature somatic cells revert to younger stem cells, and relates to regeneration *in vivo,* which occurs in insects, amphibians and plants. It does not occur naturally in mammals such as humans, but only through artificial methods. Cellular dedifferentiation methods began with methods using cell fusion. The first method of dedifferentiating somatic cells to be discovered was a method using a characteristic in which, when embryonic stem cells (ESCs) are fused with somatic cells, the embryonic stem cells become more dominant. Afterwards, research on inducing dedifferentiation of the somatic cells has progressed through fusion of somatic cells with stem cells having similar capabilities to the ESCs, for example, embryonic germinal cells (EGCs), or embryonic carcinoma cells (ECCs) in addition to the ESCs.

However, cells in which cell fusion occurs are randomly divided or maintained in a fused state without division. While a cell in which division occurs (hybrid) has one nucleus, a cell in which division does not occur (heterokaryon) has nuclei of two different cells. For this reason, most fused cells become cancer cells, and only some fused cells have normal functions.

In addition, the method of examining cell fusion (cell, cytoplasm) has a disadvantage in that many cells die due to induction of fusion using polyethylene glycol (PEG) that can damage the cells to help fusion of large-sized cells. To solve this problem, conventional research on dedifferentiating only parts of cells rather than entire embryonic stem cells has progressed. One research was performed to exclude a nucleus from a fusion by isolating only cytoplasm from a cell. A method of treating somatic cells with a chemical material (streptolysin) such that a cell membrane has permeability, and inserting cytoplasm derived from embryonic stem cells was used. However, in the case of the dedifferentiation using cytoplasm, there has been no successful case of dedifferentiation having similar characteristics to embryonic stem cells as of 2010.

In addition, there is a delivery system of a specific factor. In 2006, the Yamanaka research team of Tokyo University in Japan revealed that dedifferentiation can be performed with only four genes in embryonic stem cells. The four genes (Oct-4, klf4, Sox2, and c-Myc) were introduced into mouse skin cells, thereby preparing induced pluripotent stem cells (iPSs). This is research that began with a concept in which a part of a cell is synthesized for insertion, rather than fusion of a whole cell. The iPS technique used a method of producing a protein by inserting genes into a genome using a capability of a virus in an initial stage and compulsorily expressing RNA. However, due to the characteristics of a virus, the genes are integrated in several sites, thereby forming undesired modifications. Today, to solve this problem, an mRNA delivery method, or a method of delivery of a protein itself is used. However, it was seen that mRNA is easily degraded and has a difficult synthesis process, an immune response against RNA is induced, and the delivery of only a protein cannot achieve perfect dedifferentiation.

Accordingly, there is a demand for research on a method of preparing dedifferentiated pluripotent stem cells that can overcome the above-described problems.

As a result of research to solve the problems according to the conventional art, the inventors found that dedifferentiation of somatic cells can be effectively and stably performed using embryonic stem cell-derived microvesicles.

Accordingly, the present invention is directed to providing a method of preparing induced pluripotent stem cells by efficiently performing dedifferentiation of somatic cells with no side effects using embryonic stem cell-derived microvesicles.

However, the technical problem is not limited to the above description, and other problems not described will be clearly understood by one of ordinary skill in the art with respect to the descriptions below.

One aspect of the present invention provides a method of preparing induced pluripotent stem cells, as claimed in claim 1.

Also disclosed are induced pluripotent stem cells prepared by treating somatic cells with a composition including embryonic stem cell-derived microvesicles; and a cell therapy product including the induced pluripotent stem cells.

According to the present invention, a cytoplasm delivery method using fusion of microvesicles does not need polyethyleneglycol (PEG) needed in cell fusion or a cytotoxic material such as streptolysin used in cytoplasm injection, and thus cells are damaged less. In addition, during the cytoplasm delivery, the microvesicle has high delivery efficiency, and more effectively protects the delivered content, and thus it is possible to perform specific delivery of the cytoplasm. According to conventional research on cytoplasm delivery, cytoplasm delivery efficiency is decreased according to the amount of proteins to be expressed in a nucleus, and thus perfect dedifferentiation does not occur. However, the microvesicles of the present invention can freely control the concentration of the cytoplasm and prevent loss of intracellular materials in fusion depending on the preparation method, so that the efficiency of dedifferentiation of somatic cells using the cytoplasm can be increased.

In addition, according to the method using microvesicles of the present invention, targeting to induce *in-vivo* dedifferentiation is possible. Particularly, since a signal generated in a wounded organ of a patient is a major factor of targeting, the dedifferentiation performed *in vitro* is expected to become a new means capable of being performed *in vivo.*

### [Description of Drawings]

FIG. 1 shows transmission electron microscope (TEM) images of embryonic stem cell-derived microvesicles prepared by extrusion.
FIG. 2 is a graph showing a size of an embryonic stem cell-derived microvesicle prepared by extrusion.
FIG. 3 is a diagram showing that an embryonic stem cell-specific protein, Oct3/4, is present in embryonic stem cells and embryonic stem cell-derived microvesicles.
FIG. 4 is a diagram showing that embryonic stem cell-specific genes, Oct3/4 and Nanog, are present in embryonic stem cells and embryonic stem cell-derived microvesicles.
FIG. 5 shows colonies produced after dedifferentiation of mouse fibroblasts (NIH3T3 cells) using embryonic stem cell-derived microvesicles.
FIG. 6 shows that colonies produced after mouse fibroblasts (NIH3T3 cells) are dedifferentiated using embryonic stem cell-derived microvesicles and proliferated by lapse of time.
FIG. 7 shows colonies produced after GFP-transformed mouse fibroblasts (NIH3T3-GFP cells) are dedifferentiated using embryonic stem cell-derived microvesicles.
FIG. 8 is a diagram showing that embryonic stem cell-specific genes, Oct3/4 and Nanog, are expressed in dedifferentiated mouse fibroblasts (NIH3T3 dedifferentiated cells).
FIG. 9 shows that dedifferentiated mouse fibroblasts (NIH3T3 dedifferentiated cells) have differentiation capability.
FIG. 10 is a diagram showing that differentiation-specific genes, AFP, Foxf1, and β-III tubulin, are expressed when dedifferentiated mouse fibroblasts (NIH3T3 dedifferentiated cells) are differentiated.
FIG. 11 shows colonies produced after mouse fibroblasts (NIH3T3 cells) are dedifferentiated by treating them with embryonic stem cell-derived microvesicles in combination with brefeldin A (BFA).

The present invention provides a method of preparing induced pluripotent stem cells as claimed in claim 1.

The embryonic stem cells used in the present invention may be derived from humans, non-human primates, mice, rats, dogs, cats, horses, and cattle, but the present invention is not limited thereto.

The "microvesicle" used herein is divided into internal and external sides by a lipid bilayer composed of a cell membrane component of a derived cell, includes a cell membrane lipid, a cell membrane protein, a nucleic acid and cell components of the cell, and has a smaller size than the original one, but the present invention is not limited thereto.

The microvesicles used in the method of the present invention are prepared by extruding a suspension of embryonic stem cells. In one embodiment of the present invention, a membrane of the microvesicle may further include a component other than the cell membrane of the embryonic stem cell.

The component other than the cell membrane may include a targeting molecule, a material necessary for fusion of the cell membrane with a targeting cell (fusogen), cyclodextrin, PEG, etc. In addition, the component other than the cell membrane may be added by various methods, which include chemical modification of the cell membrane.

For example, the membrane component of the microvesicle may be chemically modified by a chemical method using a thio group (-SH) or an amine group (-NH₂), or by chemically binding PEG to the microvesicle.

The present invention may further include chemically modifying the membrane component of the microvesicle in the preparation of the microvesicle of the present invention.

The embryonic stem cell may be a transformed cell. Specifically, the transformed cell includes a cell transformed to express a material necessary for fusion of a cell membrane with a specific protein, a targeting molecule, or a target cell; and a transformed cell composed of a combination of at least two thereof, but the present invention is not limited thereto.

The embryonic stem cell may be transformed by treatment with a material or transduction, and may be transformed at least twice.

The embryonic stem cell may be transformed to inhibit expression of at least one specific protein.

The embryonic stem cell may be transformed to express at least one selected from the group consisting of a cell adhesion molecule, an antibody, a targeting protein, a cell membrane fusion protein, and a fusion protein thereof.

The embryonic stem cells may be transformed to overexpress an embryonic stem cell-specific protein, that is, Oct3/4, Nanog, or Sox-2 protein.

The embryonic stem cell-derived microvesicles may be present in a composition and the composition may further include a component other than the embryonic stem cell-derived microvesicle.

For example, the composition including the embryonic stem cell-derived microvesicle and a material stimulating dedifferentiation of somatic cells may be used to treat the somatic cells. The additional material may include brefeldin A (BFA), BiP inducer X (BIX), and valproic acid (VPA).

Hereinafter, exemplary Examples are provided to help understanding of the present invention. However, the following Examples are provided so that the present invention may be more easily understood, and the scope of the present invention is not limited to the Examples.

### Example 1. Preparation of embryonic stem cell-derived microvesicles

Mouse embryonic stem cells were resuspended in 3 ml of a phosphate buffered saline (PBS) solution at a concentration of 5 × 10⁶ cells/ml. The resuspension was passed through a membrane filter having a pore size of 10 µm 10 times, and through a membrane filter having a pore size of 5 µm 10 times. 1 ml of 50% OptiPrep™, 1 ml of 5% OptiPrep™, and 3 ml of a cell suspension passed through the membrane filter were each put in a 5 ml ultracentrifuge tube. Afterward, ultracentrifugation was performed at 100,000× g for 2 hours. A microvesicle was obtained from a layer between 50% OptiPrep™ and 5% OptiPrep™.

### Example 2. Analysis of characteristics of embryonic stem cell-derived microvesicles

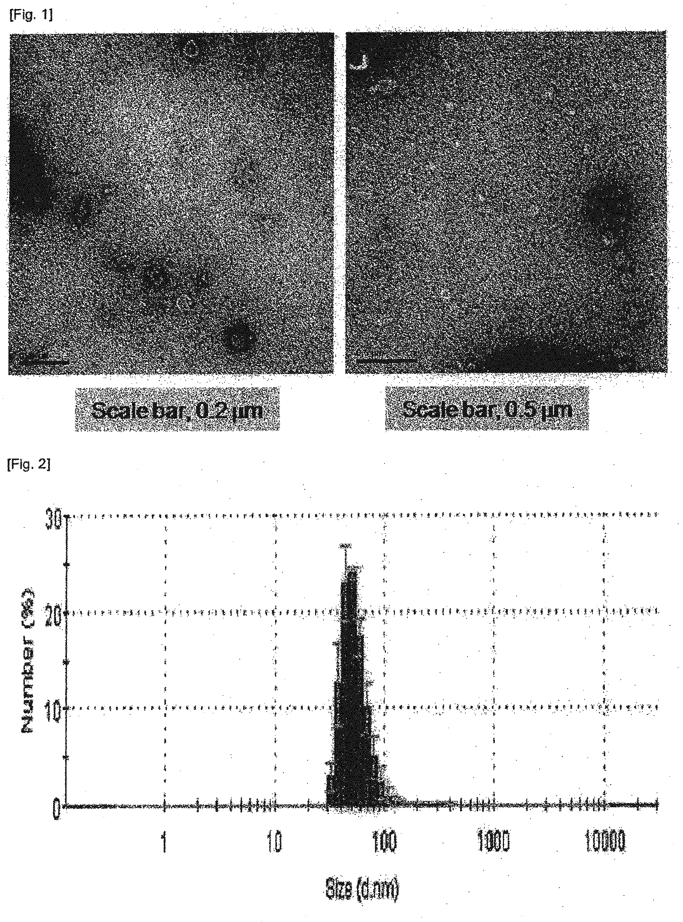

The microvesicles prepared from the embryonic stem cells according to the method described in Example 1 were adsorbed on a glow-discharged carbon-coated copper grid for 3 minutes. The grid was washed with distilled water and stained with 2% uranylacetate for 1 minute, and results observed using a transmission electron microscope, JEM101 (Jeol, Japan), are shown in FIG. 1.

As shown in the TEM images of FIG. 1, it can be seen that the microvesicle prepared by extrusion from the embryonic stem cells was composed of a lipid bilayer, and usually formed in a sphere having a size of 100 to 200 nm.

The microvesicle prepared from the embryonic stem cells described in Example 1 was diluted in 1 ml of PBS at a concentration of 5 µg/ml. 1 ml of PBS containing the microvesicles was put into a cuvette and analyzed using a dynamic light scattering particle size analyzer, and results are shown in FIG. 2.

As shown in FIG. 2, it is confirmed that the microvesicle had a size of 50 to 100 nm, and an average size of 70 nm.

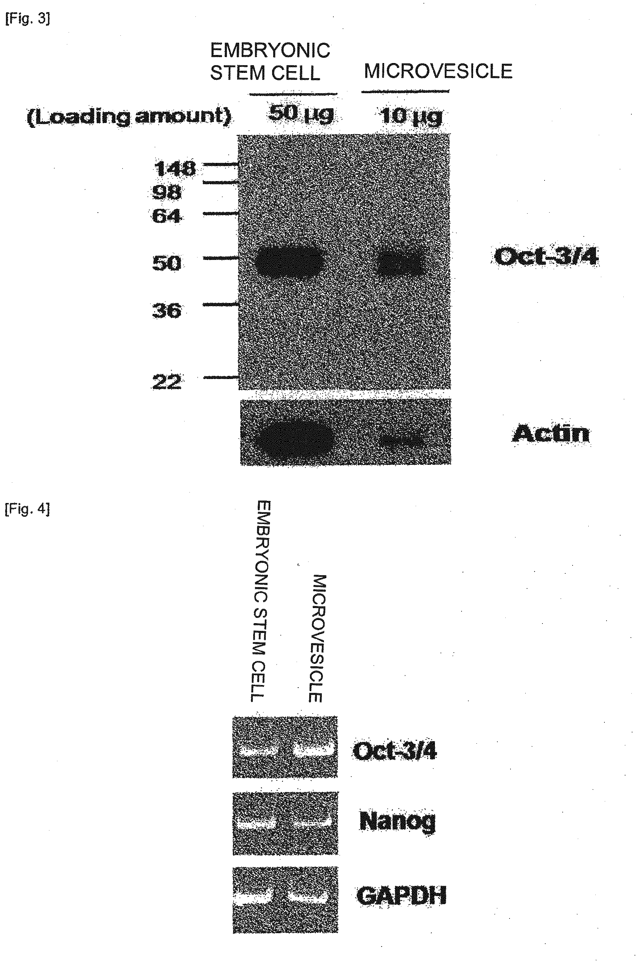

50 µg of the microvesicles prepared from the embryonic stem cells according to the method described in Example 1 and 10 µg of a whole cell lysate of the embryonic stem cells were prepared, a 5x loading dye (250 mM Tris-HCl, 10% SDS, 0.5% bromophenol blue, 50% glycerol) was added to finally become 1x, and the resulting solution was treated at 100 °C for 5 minutes. 8% polyacrylamide gel was prepared, and then a sample was loaded. The sample was subjected to electrophoresis at 80 V for 2 hours, and a protein was transferred to a polyvinylidene fluoride (PVDF) membrane at 400 mA for 2 hours. Skim milk was dissolved in PBS to have a concentration of 3%, and the membrane was blocked in the solution for 2 hours. Oct3/4 and β-actin antibodies were treated at 4 °C for 12 hours. The resulting membrane was washed with PBS twice, and secondary antibodies to which a peroxidase was attached were treated at room temperature for 1 hour. The resulting membrane was washed with PBS for 30 minutes and identified using an enhanced chemiluminescence (ECL; Amersham Co. No. RPN2106) substrate, results of which are shown in FIG. 3.

As shown in FIG. 3, it is confirmed that an embryonic stem cell-specific protein, that is, Oct3/4, was present in the microvesicles prepared from the embryonic stem cells.

Total genes (RNAs) were extracted from the microvesicles prepared from the embryonic stem cells according to the method described in Example 1 and the embryonic stem cells using an RNeasy®Mini kit (QIAGEN, Cat. No. 74104). A plurality of cDNAs were obtained from the extracted RNA using a specific gene primer and a PCR kit (BIOLAB, Cat. No. E5000), isolated by agarose gel electrophoresis, and identified using ethidium bromide (ETBR) staining. To confirm that the same amount of RNA was used, a positive control (embryonic stem cells) was used; a housekeeping gene (glyceraldehyde 3-phosphate dehydrogenase, GAPDH) was also tested. Here, as a sense primer for an embryonic stem cell-specific gene, that is, Oct-3/4, 5'-AGACCATGTTTCTGAAGTGC-3' was used, and as an antisense primer thereof, 5'-GAACCATACTCGAACCACA-3', was used. As a sense primer for another embryonic stem cell-specific gene, that is, Nanog, 5'-CTAGTTCTGAGGAAGCATCG-3' was used, and as an antisense primer thereof, 5'-TCTCAGTAGCAGACCCTTG-3' was used. For analysis of characteristics of the embryonic stem cell-derived microvesicles, gene expression images are shown in FIG. 4.

As shown in FIG. 4, it is confirmed that embryonic stem cell-specific genes, Oct3/4 and Nanog, were expressed in the microvesicles prepared from the embryonic stem cells.

### Example 3. Dedifferentiation of somatic cells using embryonic stem cell-derived microvesicles

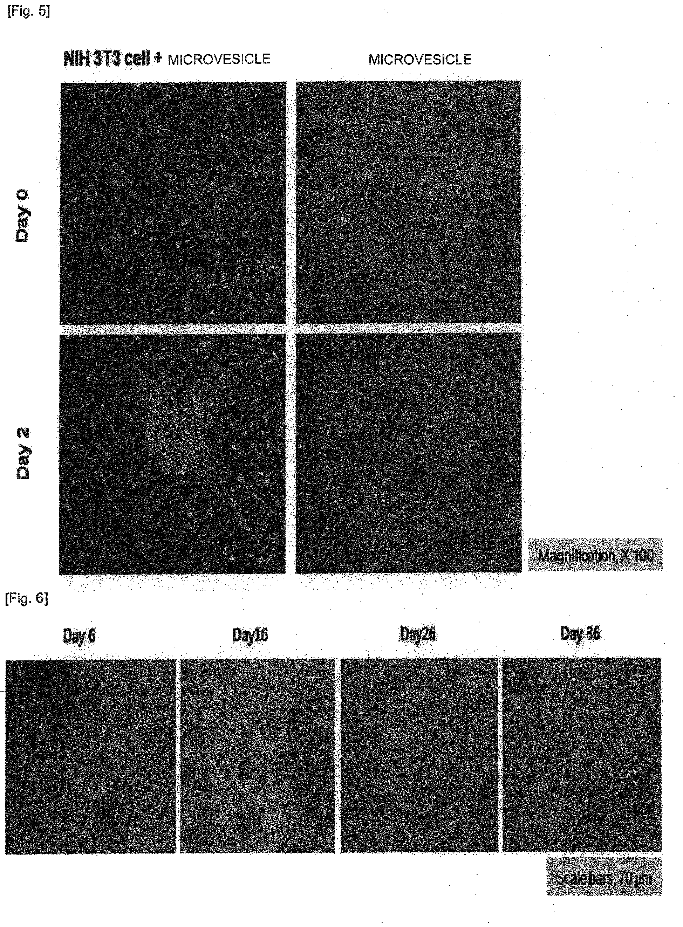

0.1% gelatin was coated on a 6-well plate and inoculated with 8×10⁴ of NIH3T3 cells, and the cells were incubated for 24 hours. Afterward, each well was washed with PBS, 2 ml of the microvesicles prepared from the embryonic stem cells according to the method described in Example 1 were diluted in a fibroblast medium (DMEM, 10% FBS, 100 U/ml penicillin-streptomycin) at a concentration of 100 µg/ml, and then used to treat the incubated NIH3T3 cells. After 48 hours, approximately 2 to 3 colonies per well were identified, and each colony had a size of approximately 10 to 100 µm. The colonies were observed using an electron microscope, and results are shown in FIG. 5.

As shown in FIG. 5, it is confirmed that the NIH3T3 cells were dedifferentiated using the embryonic stem cell-derived microvesicles, thereby inducing colonies. Each well was washed with PBS, and 400 µl of 0.1× TE (Typsin-EDTA) was added. After 1 minute, 10 µl of 1x TE was added to the colony using a pipette, and sucked up by placing the tip of the pipette to surround the colony. The picked up colonies were diluted in 500 µl of an embryonic stem cell medium (knock-out DMEM, 15% knock-out FBS, 10 ng/ml LIF, 0.1 mM 2-mercaptoethanol, 4 mM L-glutamine, 10 µg/ml of gentamycin, 100 U/ml penicillin-streptomycin), inoculated into a 0.1% gelatin-coated 24-well plate, and incubated for 5 days or more.

At the point of time at which the cells had grown to 80% confluency or more, whole somatic cells were diluted in 2 ml of the embryonic stem cell medium using 1× TE, and subcultured in a 0.1% gelatin-coated 6-well plate. Through the same method as described above, at the point of time at which the cells had grown to 80% confluency, the cells were diluted in 8 ml of a medium, subcultured in a 0.1% gelatin-coated 100 mm culture dish, and subcultured again every 2 to 3 days.

As shown in FIG. 6, it is confirmed from 5 or more days of observation that colonies were continuously proliferated.

### Example 4. Confirmation of origin of induced pluripotent stem cells

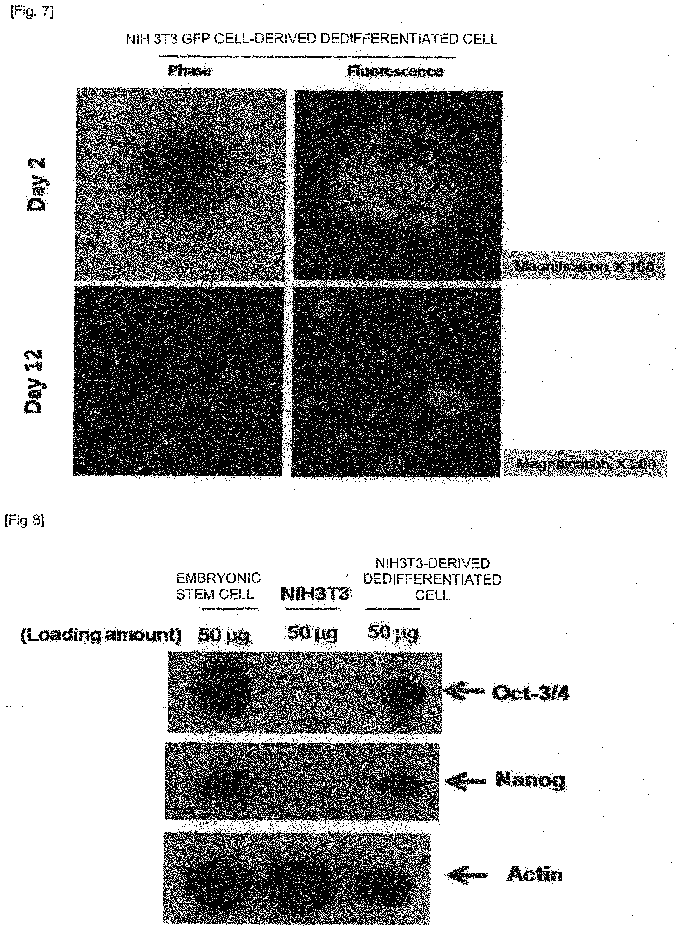

A GFP gene was injected into a genome of an NIH3T3 cell using a retrovirus (pMSCV). The transformed cell exhibited green fluorescence, but the embryonic stem cells used in forming microvesicles did not exhibit fluorescence. A 6-well plate was coated with 0.1% gelatin and inoculated with 8×10⁴ of NIH3T3 GFP cells, and the cells were incubated for 24 hours. Afterward, each well was washed with PBS, and the embryonic stem cell-derived microvesicles prepared in Example 1 were diluted in 2 ml of a fibroblast medium (DMEM,
10% FBS, 100 U/ml penicillin-streptomycin) at a concentration of 100 µg/ml and used to treat the NIH3T3 GFP cell. After 48 hours, approximately 2 to 3 colonies per well were identified, and the size of the colonies was approximately 10 to 100 µm, and results observed by an electron microscope are shown in FIG. 7.

According to the conventional research, a long period, for example, at least 7 to 30 days, of dedifferentiation was needed, but, as shown in FIGS. 6 and 7, in the case of the embryonic stem cell-derived microvesicles of the method of the present invention, it can be seen that colonies were formed in only 48 hours, and thus the period of dedifferentiation could be significantly reduced.

### Example 5. Confirmation of characteristics of induced pluripotent stem cells

Colonies (NIH3T3-derived dedifferentiation cells) proliferated over 40 days according to the method described in Example 3, embryonic stem cells, and a whole cell lysate of the NIH3T3 cells were quantified by a detergent compatible protein assay (DC), and thus 50 µg of each was prepared, and a 5x loading dye was added to have a final concentration of 1x, and treated at 100 °C for 5 minutes. 8% polyamide gel was prepared, and a sample was loaded. The sample was subjected to electrophoresis at 80 V for 2 hours, and a protein was transferred to a PVDF membrane at 400 mA for 2 hours. The membrane used different blocking buffers according to the antibodies to be applied. In the case of actin, skim milk was dissolved in PBS to have a concentration of 3%, and in the case of Oct 3/4, non fat dry milk was dissolved in PBS to have a concentration of 5%, in the case of Nanog, 10% non fat dry milk was dissolved in PBS to have a concentration of 10%, and the membrane was blocked in this solution for 2 hours. Oct3/4, Nanog, and β-actin antibodies were treated at 4 °C for 12 hours, the membrane was washed with PBS twice, and secondary antibodies to which a peroxidase was attached were treated at room temperature for 1 hour. The resulting membrane was washed with PBS for 30 minutes, and results were identified using an ECL substrate, which are shown in FIG. 8.

As shown in FIG. 8, it is confirmed that embryonic stem cell-specific proteins, Oct3/4 and Nanog, were expressed in NIH3T3 dedifferentiation cells (induced pluripotent stem cells) dedifferentiated by embryonic stem cell-derived microvesicles.

### Example 6. Confirmation of spontaneous differentiation of induced pluripotent stem cells

To identify a function of the induced pluripotent cells proliferated over 40 days by the method described in Example 3, spontaneous differentiation capability to all cells was confirmed. When the embryonic stem cells became an embryonic body, the embryonic body had a capability to be spontaneously differentiated to all cells. By the same principle, cells induced by a hanging drop method were induced to compulsorily agglomerate. Colony cells were suspended using trypsin, and diluted in a differentiation medium (IMDM, 20% FBS, 4 mM L-glutamine, 10 µg/ml gentamycin, 100 U/ml penicillin-streptomycin) at a concentration of 3.3 × 10⁴/ml. 30 µl (1 × 10³) of the diluted solution was attached to a top surface of a bacteria culture dish, and induced to agglomerate for 2 days. The cells agglomerating on the top surface were washed with a differentiation medium to be resuspended in a bacteria culture dish, and incubated for 2 days. FIG. 8 is images of agglomerated induced pluripotent stem cells suspended in the bacteria culture dish. By the same method, NIH3T3 cells and embryonic stem cells were induced to agglomerate by a hanging drop method.

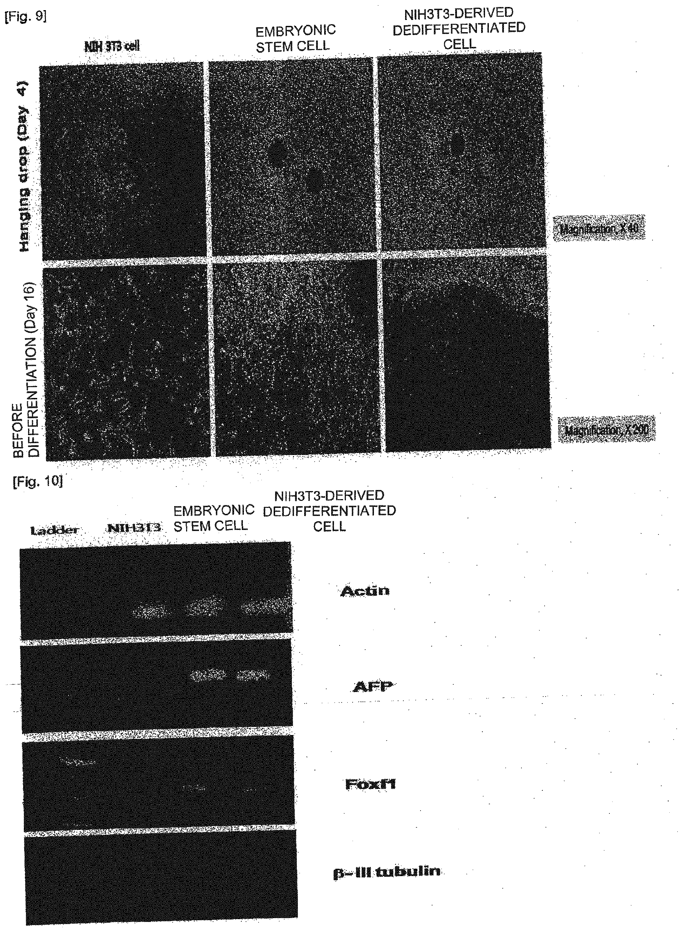

An embryonic body was diluted in a differentiation medium to have a confluency of 3/ml, and then 2 ml of the diluted body was injected into each well of a 0.1% gelatin-coated 6-well plate to incubate. A fresh culture solution was transferred twice, that is, every 8 days for 16 days. FIG. 9 shows the cells after induction to differentiation.

Total genes (RNAs) were extracted from the differentiated cells using an RNeasy®Mini kit (QIAGEN, Cat. No. 74104). A plurality of cDNAs were obtained from the extracted RNAs using a specific gene primer and a PCR kit (BIOLAB, Cat. No. E5000), isolated by agarose gel electrophoresis, and identified by ETBR staining. To confirm that the same amount of RNA was used, a positive control (embryonic stem cells) was used; a housekeeping gene (actin) was also tested. As a result of confirming the spontaneous differentiation of the induced pluripotent stem cells, it was confirmed that the cells were differentiated into an endoderm (AFP), a mesoderm (Foxf1), and an ectoderm (b-III tubulin) of NIH3T3 dedifferentiation cells. For AFP, 5'-AACTCTGGCGATGGGTGTT-3' was used as a sense primer and 5'-AAACTGGAAGGGTGGGACA-3' was used as an antisense primer. For Foxf1, 5'-CGTGTGTGATGTGAGGTGAG-3' was used as a sense primer and 5'-CTCCGTGGCTGGTTTCA-3' was used as an antisense primer. For b-III tubulin, 5'-TTTTCGTCTCTAGCCGCGTG-3' was used as a sense primer and 5'-GGCCCTGGGCACATACTTGTG-3' was used as an antisense primer. FIG. 10 shows images for checking whether the gene is expressed to confirm a spontaneous differentiation capability of NIH3T3 dedifferentiation cells (induced pluripotent stem cells).

As shown in FIG. 10, it is confirmed that endodermal, mesodermal, and ectodermal genes were expressed in the NIH3T3 dedifferentiation cells, which shows that the NIH3T3 dedifferentiation cells have a spontaneous differentiation capability.

### Example 7. Dedifferentiation of somatic cells according to simultaneous treatment of embryonic stem cell-derived microvesicles and BFA

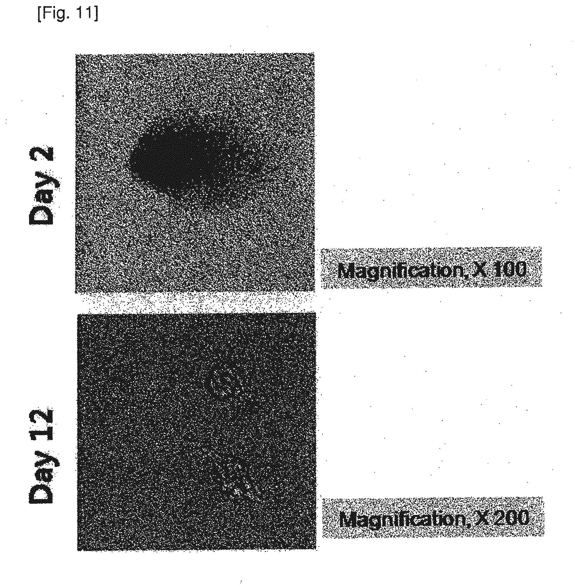

0.1% gelatin was coated on a 6-well plate, and 8×10⁴ of NIH3T3 cells were inoculated into each well and incubated for 24 hours. After the well was washed with PBS, the embryonic stem cell-derived microvesicles prepared in Example 1 were diluted in 2 ml of a fibroblast medium (DMEM, 10% FBS, 100 U/ml penicillin-streptomycin) at a concentration of 100 µg/ml, and BFA was diluted in 2 ml of a fibroblast medium (DMEM, 10% FBS, 100 U/ml penicillin-streptomycin) at a concentration of 2 µM, and then each of the resulting products was used to treat the incubated NIH3T3 cells. After 48 hours, 2 to 3 colonies per well were identified, and the size of the colonies was approximately 10 to 100 µm. Results observed by an electron microscope are shown in FIG. 11.

As shown in FIG. 11, it is confirmed that colonies could be induced even when embryonic stem cell-derived microvesicles and BFA were simultaneously treated.

Each well was washed with PBS, and 400 µl of 0.1× TE (Typsin-EDTA) was added. After 1 minute, 10 µl of 1x TE was added to the colony using a pipette, and sucked up by placing the tip of the pipette to surround the colony. The picked up colonies were diluted in 500 µl of an embryonic stem cell medium (knock-out DMEM, 15% knock-out FBS, 10 ng/ml LIF, 0.1 mM 2-mercaptoethanol, 4 mM L-glutamine, 10 µg/ml gentamycin, 100 U/ml penicillin-streptomycin), inoculated into a 0.1% gelatin-coated 24-well plate, and incubated for 5 days or more.

As shown in FIG. 11, it is confirmed that the colonies were continuously proliferated.

Since a method of preparing induced pluripotent stem cells according to the present invention uses a cytoplasm delivery method using fusion of microvesicles, it is expected that dedifferentiation of somatic cells can be efficiently performed without side effects, and the method can be effectively used to develop a cell therapy product having different immunocompatibilities for different individuals.

## Claims

1. A method of preparing induced pluripotent stem cells, the method comprising the steps:
(i) extruding a suspension of embryonic stem cells to produce embryonic stem cell-derived microvesicles;
(ii) dedifferentiating somatic cells using the embryonic stem cell-derived microvesicles, to produce induced pluripotent stem cells,
whereby the resultant induced pluripotent stem cells have a spontaneous differentiation capability to all cells.

2. The method according to claim 1, wherein the embryonic stem cell is derived from one selected from the group consisting of a human, a non-human primate, a mouse, a rat, a dog, a cat, a horse, and cattle.

3. The method according to claim 1, wherein the embryonic stem cell is a transformed cell.

4. The method according to claim 1, wherein the embryonic stem cell is a cell transformed to overexpress Oct3/4, Nanog, or Sox-2 protein as an embryonic stem cell-specific protein.

5. The method according to claim 1, wherein the embryonic stem cell is a cell transformed to express at least one selected from the group consisting of a cell adhesion molecule, an antibody, a targeting protein, a cell membrane fusion protein, and a fusion protein thereof.

6. The method according to claim 1, wherein a membrane of the microvesicle further includes a component other than a cell membrane of the embryonic stem cell.

7. The method according to claim 6, wherein the component other than the cell membrane is cyclodextrin or polyethyleneglycol.

8. The method according to claim 1, wherein the membrane component of the microvesicle is chemically modified.

9. The method according to claim 1, wherein the embryonic stem cell-derived microvesicles are present in a composition and the composition further includes a component other than the embryonic stem cell-derived microvesicle.

10. The method according to claim 9, wherein the component other than the embryonic stem cell-derived microvesicle is brefeldin A (BFA), BiP inducer X (BIX), or valproic acid (VPA).

## Patentansprüche

1. Verfahren zur Herstellung von induzierten pluripotenten Stammzellen, wobei das Verfahren die Schritte umfasst:
(i) Extrudieren einer Suspension von embryonalen Stammzellen um von embryonalen Stammzellen abgeleitete Mikrovesikel herzustellen;
(ii) Entdifferenzieren somatischer Zellen unter Verwendung der von embryonalen Stammzellen abgeleiteten Mikrovesikel , um induzierte pluripotente Stammzellen herzustellen,
wobei die resultierenden induzierten pluripotenten Stammzellen eine spontane Differenzierungsfähigkeit zu allen Zellen aufweisen.

2. Verfahren nach Anspruch 1, wobei die embryonale Stammzelle von einem, ausgewählt aus der Gruppe, bestehend aus einem Menschen, einem nichtmenschlichen Primaten, einer Maus, einer Ratte, einem Hund, einer Katze, einem Pferd und Rind, abgeleitet ist.

3. Verfahren nach Anspruch 1, wobei die embryonale Stammzelle eine transformierte Zelle ist.

4. Verfahren nach Anspruch 1, wobei die embryonale Stammzelle eine Zelle ist, die transformiert ist, um Oct3/4, Nanog oder Sox-2-Protein als ein embryonales Stammzellspezifisches Protein zu überexprimieren.

5. Verfahren nach Anspruch 1, wobei die embryonale Stammzelle eine Zelle ist, die transformiert ist, um mindestens eines ausgewählt aus der Gruppe, bestehend aus einem Zelladhäsionsmolekül, einem Antikörper, einem Targeting-Protein, einem Zellmembran-Fusionsprotein und einem Fusionsprotein davon, zu exprimieren.

6. Verfahren nach Anspruch 1, wobei eine Membran des Mikrovesikels weiter eine andere Komponente als eine Zellmembran der embryonalen Stammzelle enthält.

7. Verfahren nach Anspruch 6, wobei die andere Komponente als die Zellmembran Cyclodextrin oder Polyethylenglykol ist.

8. Verfahren nach Anspruch 1, wobei die Membrankomponente des Mikrovesikels chemisch modifiziert wird.

9. Verfahren nach Anspruch 1, wobei die von embryonalen Stammzellen abgeleiteten Mikrovesikel in einer Zusammensetzung vorliegen und die Zusammensetzung weiter eine andere Komponente als das von embryonalen Stammzellen abgeleitete Mikrovesikel enthält.

10. Verfahren nach Anspruch 9, wobei die andere Komponente als das von embryonalen Stammzellen abgeleitete Mikrovesikel Brefeldin A (BFA), BiP-Inducer X (BIX) oder Valproinsäure (VPA) ist.

## Revendications

1. Procédé de préparation de cellules souches pluripotentes induites, le procédé comprenant les étapes de :
(i) extruder une suspension de cellules souches embryonnaires pour produire des microvésicules dérivées de cellules souches embryonnaires ;
(ii) dédifférencier des cellules somatiques en utilisant les microvésicules dérivées de cellules souches embryonnaires, pour produire des cellules souches pluripotentes induites,
de sorte que les cellules souches pluripotentes induites résultantes ont une capacité de différenciation spontanée en toutes cellules.

2. Procédé selon la revendication 1, dans lequel la cellule souche embryonnaire est dérivée d'une cellule choisie dans le groupe consistant en un humain, un primate non humain, une souris, un rat, un chien, un chat, un cheval et du bétail.

3. Procédé selon la revendication 1, dans lequel la cellule souche embryonnaire est une cellule transformée.

4. Procédé selon la revendication 1, dans lequel la cellule souche embryonnaire est une cellule transformée pour surexprimer la protéine Oct3/4, Nanog ou Sox-2 en tant que protéine spécifique de cellule souche embryonnaire.

5. Procédé selon la revendication 1, dans lequel la cellule souche embryonnaire est une cellule transformée pour exprimer au moins une cellule choisie dans le groupe consistant en une molécule d'adhésion cellulaire, un anticorps, une protéine de ciblage, une protéine de fusion de membrane cellulaire et une protéine de fusion de celle-ci.

6. Procédé selon la revendication 1, dans lequel une membrane de la microvésicule inclut en outre un composant autre qu'une membrane cellulaire de la cellule souche embryonnaire.

7. Procédé selon la revendication 6, dans lequel le composant autre que la membrane cellulaire est la cyclodextrine ou le polyéthylèneglycol.

8. Procédé selon la revendication 1, dans lequel le composant membranaire de la microvésicule est chimiquement modifié.

9. Procédé selon la revendication 1, dans lequel les microvésicules dérivées de cellules souches embryonnaires sont présentes dans une composition et la composition inclut en outre un composant autre que la microvésicule dérivée de cellules souches embryonnaires.

10. Procédé selon la revendication 9, dans lequel le composant autre que la microvésicule dérivée de cellules souches embryonnaires est la brefeldine A (BFA), l'inducteur BiP X (BIX) ou l'acide valproïque (VPA).
